# EUROPEAN PATENT SPECIFICATION

(11) **EP 1 964 563 B1**
(45) Date of publication and mention of the grant of the patent: **10.09.2014**
(21) Application number: 06783799.7
(22) Date of filing: 12.07.2006
(51) Int. Cl.: A61K 31/4422, A61K 45/06, A61K 31/616, A61P 9/10

(54) **PHARMACEUTICAL COMPOSITION COMPRISING AMLODIPINE AND ACETYLSALICYLIC ACID FOR USE IN THE TREATMENT OF SYSTEMIC ARTERIAL HYPERTENSION**
PHARMAZEUTISCHE ZUSAMMENSETZUNG ENTHALTEND AMLODIPIN UND ACETYLSALICYLSÄURE ZUR VERWENDUNG ZUR BEHANDLUNG VON SYSTEMISCHER ARTERIELLER HYPERTONIE
COMPOSITION PHARMACEUTIQUE COMPRENANT DE L'AMLODIPINE ET DE L'ACIDE ACÉTYLSALICYLIQUE ET SON UTILISATION POUR LE TRAITEMENT DE L'HYPERTENSION ARTÉRIELLE SYSTÉMIQUE

(30) Priority: 20.12.2005 MX PA05014087
(43) Date of publication of application: 03.09.2008
(73) Proprietor: PPTM INTERNATIONAL S.à r.l., 1253 Luxembourg (LU)
(72) Inventor: GARCÍA ARMENTA,María, Elena, C.P. 45020 Guadalajara, Jalisco (MX); ÁLVAREZ OCHOA, Víctor, Guillermo, C.P. 45222 Zapopan, Jalisco (MX); SANTOS MURILLO, Josefina, C.P. 44600 Zapopan, Jalisco (MX); GARCÍA ARMENTA, Patricia, C.P. 45110 Zapopan, Jalisco (MX)
(74) Representative: Carvajal y Urquijo, Isabel
(86) International application number: PCT/MX2006/000069
(87) International publication number: WO 2007/073132

(56) References cited:
- WO-A-01/76632
- WO-A-2004/080488
- US-A1- 2003 099 724
- FOLTS J.D.: 'Inhibition of platelet activity in vivo by amlodipine alone and combined with aspirin' INTERNATIONAL JOURNAL OF CARDIOLOGY vol. 62, no. SUPPL. 2, 1997, pages S111 - S117, XP003014741
- DATABASE BIOSIS [Online] BIOSCIENCES INFORMATION SERVICE, PHILADELPHIA, PA, US 1988 FRISHMAN W H ET AL: 'A RANDOMIZED PLACEBO-CONTROLLED COMPARISON OF AMLODIPINE AND ATENOLOL IN MILD TO MODERATE SYSTEMIC HYPERTENSION' Database accession no. PREV198936087988 & JOURNAL OF CARDIOVASCULAR PHARMACOLOGY, vol. 12, no. SUPPL. 7, 1988, pages S103-S106, SATELLITE SYMPOSIUM ON CALCIUM ANTAGONISTS IN CARDIOVASCULAR DISEASE: RATIONALE FOR 24-HOUR ACTION H ISSN: 0160-2446
- DATABASE EMBASE [Online] ELSEVIER SCIENCE PUBLISHERS, AMSTERDAM, NL 1988 MACLEAN D ET AL: 'Amlodipine and captopril in moderate-severe essential hypertension' Database accession no. EMB-1988219310 & MACLEAN D ET AL: "Amlodipine and captopril in moderate-severe essential hypertension", JOURNAL OF HUMAN HYPERTENSION 1988 GB, vol. 2, no. 2, 1988, pages 127-132, ISSN: 0950-9246
- DATABASE BIOSIS [Online] BIOSCIENCES INFORMATION SERVICE, PHILADELPHIA, PA, US 1989 FRICK M H ET AL: 'A DOSE-RESPONSE STUDY OF AMLODIPINE IN MILD TO MODERATE HYPERTENSION' Database accession no. PREV198988077335 & FRICK M H ET AL: "A DOSE-RESPONSE STUDY OF AMLODIPINE IN MILD TO MODERATE HYPERTENSION", JOURNAL OF INTERNAL MEDICINE, vol. 225, no. 2, 1989, pages 101-106, ISSN: 0954-6820
- DATABASE BIOSIS [Online] BIOSCIENCES INFORMATION SERVICE, PHILADELPHIA, PA, US 1993 MEHTA JAWAHAR L ET AL: 'Double-blind evaluation of the dose-response relationship of amlodipine essential hypertension' Database accession no. PREV199396067772 & MEHTA JAWAHAR L ET AL: "Double-blind evaluation of the dose-response relationship of amlodipine essential hypertension", AMERICAN HEART JOURNAL, vol. 125, no. 6, 1993, pages 1704-1710, ISSN: 0002-8703
- DATABASE BIOSIS [Online] BIOSCIENCES INFORMATION SERVICE, PHILADELPHIA, PA, US 1996 COREA LUIGI ET AL: 'Valsartan, a new angiotensin II antagonist for the treatment of essential hypertension: A comparative study of the efficacy and safety against amlodipine' Database accession no. PREV199699250877 & COREA LUIGI ET AL: "Valsartan, a new angiotensin II antagonist for the treatment of essential hypertension: A comparative study of the efficacy and safety against amlodipine", CLINICAL PHARMACOLOGY AND THERAPEUTICS, vol. 60, no. 3, 1996, pages 341-346, ISSN: 0009-9236
- DATABASE BIOSIS [Online] BIOSCIENCES INFORMATION SERVICE, PHILADELPHIA, PA, US June 1998 TIEBEL RENATE ET AL: 'Efficacy, safety, and effects on hypertension-associated symptoms of losartan, alone or in combination with hydrochlorothiazide, versus amlodipine in patients with mild-to-moderate hypertension' Database accession no. PREV199800351519 & TIEBEL RENATE ET AL: "Efficacy, safety, and effects on hypertension-associated symptoms of losartan, alone or in combination with hydrochlorothiazide, versus amlodipine in patients with mild-to-moderate hypertension", CURRENT THERAPEUTIC RESEARCH, vol. 59, no. 6, June 1998 (1998-06), pages 325-340, ISSN: 0011-393X
- DATABASE BIOSIS [Online] BIOSCIENCES INFORMATION SERVICE, PHILADELPHIA, PA, US October 2000 DE LUCA NICOLA ET AL: 'Haemodynamic and metabolic effects of rilmenidine in hypertensive patients with metabolic syndrome X. A double-blind parallel study versus amlodipine' Database accession no. PREV200000517495 & DE LUCA NICOLA ET AL: "Haemodynamic and metabolic effects of rilmenidine in hypertensive patients with metabolic syndrome X. A double-blind parallel study versus amlodipine", JOURNAL OF HYPERTENSION, vol. 18, no. 10, October 2000 (2000-10), pages 1515-1522, ISSN: 0263-6352

## Description

### FIELD OF THE INVENTION

The present invention relates to the pharmaceutical industry in general and, in particular, to the pharmaceutical industry preparer of medicaments for the control of Systemic Arterial Hypertension and prevention of ischemic vascular events.

### BACKGROUND OF THE INVENTION

Over the last years, complications derived from heart diseases gave rise to a 15.5 percent of total deceases registered in the country, and it continues to increase since their prevalence in this heading is of 71.1 per each 100 thousand inhabitants, so these ailments are considered the leader cause of death.

It is particularly in the last two decades when the deceases originated by conditions such as coronary insufficiency, ischemic heart disease and hypertensive disease, among others, have shown an increasing tendency such that its increase during that period has been of 33.1 percent.

Systemic arterial hypertension (SAH) is probably the most common chronic degenerative disease in both, Mexico and Latin America. Its prevalence ranges from 10 to 30% of the adult population and its presence increases with the patients' age.

In people older than 60 years old, SAH has become a significant risk factor for the presence of diseases, mainly those of cardiac and cerebrovascular origin. Patients older than 65 years old are often in risk of suffering arterial hypertension without being aware of such fact and, therefore, are exposed to its complications.

During the 2000 National Health Survey (NHS) applied in all of the country to 38 thousand 377 people with an age ranging from 20 to 69 years old, the results concluded that 15.2 of Mexican people suffer from systemic arterial hypertension (SAH) and that 61 percent were unaware of their hypertensive condition. These amounts mean that five out of ten Mexican people older than 50 years old suffer such disease and, each year, about 1.5 percent of them are in risk of death due to causes directly related to such hypertension.

Arterial hypertension (SAH) could be defined as a varying increase of mechanical and neurohumoral load over cardiovascular system, responsible for various, acute and chronic, degenerative processes that affect heart and arteries. The structural and functional alterations of heart and the vascular system, related to SAH, are those determining its sociosanitary importance. There is a pluripotentiality of pathogenetic mechanisms involved in cardiovascular disease of the hypertensive patient, so the treatment should not only pursue the normalization of tension, but also prevent or limit the cardiovascular pathology associated with SAH.

Patients with hypertension die prematurely, being cardiac affection, ictus, and renal insufficiency the most common causes.

Most of these cardiac problems can lead to acute myocardial infarction, which in 48 percent of cases has as an imminent first alarm signal the experiencing of acute pain in chest, called angina, caused by high cholesterol, arterial hypertension, diabetes and tobacco smoking.

It is estimated that more than one million 500 thousand cases of unstable angina pectoris and myocardial infarctions occur worldwide every year.

Angina pectoris is an episodic clinical syndrome due to transient myocardial ischemia. Males constitute about 70% of all patients with angina and an even higher percentage among patients younger than 50 years old.

Inadequate oxygenation induced by coronary atherosclerosis can lead to transient alterations of mechanical, biochemical and electrical function of myocardium. The abrupt development of ischemia usually affects the myocardium in its left ventricle with almost instant failure of normal contraction and relaxation of muscle.

The relatively deficient perfusion of subendocardium produces a more intense ischemia in this portion of the wall. Ischemia of large segments of ventricle causes transient heart failure and, if the papillary muscles are affected, the mitral insufficiency can complicate the episode. When the episodes are transient, these can be associated to angina pectoris: If these are extended, then they cause necrosis and wound healing of myocardium with or without the acute myocardial infarction clinical chart.

Coronary atherosclerosis is a localized process causing irregular ischemia. As a consequence, the regional alterations of myocardial contractility give rise to protrusions or segmental dyskinesia and can considerably decrease the effectiveness of myocardial pump function.

*Stable angina pectoris*: is characterized by the presence of pain clearly related to effort and/or cold, of retrosternal location with irradiation to jaw and, in a less specific fashion, to arms. That Angor which has modified neither its intensity nor prematurity to appear during the last month it is considered stable. Such type of angina attributes to atherosclerotic lesions associated neither to ulcers nor to thrombus, which predict an imminent infarction. As noted above; with age, the Angor progressively losses its typical character, becoming its association of appearance with effort or at the retrosternal site less evident.

Platelet activation performs a crucial role in the genesis of acute coronary charts determined by the rupture of acute atherosclerotic plaque.

The formation of blood platelet thrombus performs a fundamental role in the pathogenesis of acute coronary symptoms (ACS), including unstable angina (UA), non Q-wave myocardial infarction (NQMI) and Q-wave myocardial infarction and ST-elevation.

Platelet adhesion, activation and aggregation processes take part in the formation of blood platelet thrombus. When endothelial damage is produced, the adhesion of circulating platelets to ligands of the damaged vascular wall begin, such as the exposed collagen, vitronectin, von Willebrand factor and thrombin. The adhesion is followed by the platelet activation, through multiple mechanisms involving various agonists, including collagen, thrombin, adenosine diphosphate (ADP), thromboxane A2, serotonin, noradrenalin and adrenalin, many of which are found in high concentrations at the vascular lesion location. As a consequence of activation, platelet degranulation is produced, releasing more ADP, thromboxane A2 and generating more thrombin from the circulating prothrombin. All of which increases the extent of platelet activation, and recruits a higher number of platelets to the thrombus scenario.

Platelet activation induces a conformational change on the platelet, which carries the exposure and activation of the platelet receptors IIb/IIIa of GP stored within the platelet, to the surface thereof. Once activated, the receptor IIb/IIIa binds to fibrinogen, which is its primary ligand, the platelets aggregated and form a real thrombus.

The conversion of fibrinogen into fibrin by the thrombin will finally stabilize the thrombus. The binding of fibrinogen to receptor IIb/IIIa is the only mediator of platelet aggregation and is independent from platelet activation mechanisms. Therefore, the binding of the fibrinogen to receptor IIb/IIIa has been denoted the common final via in blood platelet thrombus formation.

The receptor of GP, IIb/IIIa, belongs to the family of integrins. It is the most abundant protein on the platelet surface, with 50,000 to 80,000 copies; another reserve maintains in the intracellular stocks. It is composed by two protein units: subunit a (IIb), made up of an extracellular heavy chain and of another light chain with 3 segments located in cytoplasm, cellular membrane and extracellular level, respectively; and by subunit b (IIIa), made up of a single chain with an intracytoplasmic tail, a transmembranal segment and another located outside the cell.

The integral treatment in patients with Systemic Arterial Hypertension includes pharmacological and non-pharmacological measures with the purpose of decreasing cardiovascular alterations risks in these patients; it is important to mention that, joined to antihypertensive therapy, the association of platelet aggregation inhibiting medicaments with the purpose of additionally decreasing the risks of ischemic vascular events is common, so it is of interest to review the calcium channel blockers as well as the platelet aggregation inhibitors.

Calcium antagonists are first-choice drugs for the treatment of SAH; these are potent anti-anginal agents that do not cause metabolic alterations. They are classified in dihydropiridinics, with higher vascular selectivity and less pressor effect like amlodipine.

### AMLODIPINE

Amlodipine is an inhibitor of calcium ion intake (slow channel blocker or calcium ion antagonist) and inhibits the transmembrane effluence of calcium ions in both cardiac muscle and smooth muscle. The action mechanism of amlodipine is due to the direct relaxing effect over the smooth muscle of vessels. The precise mechanism through which amlodipine relieves angina has not completely been clarified, but amlodipine reduces the total ischemic load by means of the two following actions:
1. Amlodipine dilates peripheral arterioles, decreasing the total peripheral resistance (post-load) against which the heart works. As the heart rate is not modified, this decrease in cardiac workload comes with a decrease in both energy consumption and oxygen requirements by the myocardium.

The action mechanism of amlodipine probably also involves dilatation of the main coronary arteries and arterioles in both ischemic and normal regions.

Such dilatation increases oxygen input to myocardium in patients with coronary arterial spasm (varying angina or Prinzmetal's angina) and in acute coronary vasoconstriction episodes.

### ACETYL SALICYLIC ACID

Acetyl salicylic acid induces a long-term functional effect on platelets, which can be clinically detected as a bleeding time extension. This effect is mainly due to irreversible inactivation of the enzyme key in the arachidonic acid platelet metabolism. This enzyme, prostaglandin H-synthetase, is responsible for the formation of PGH₂, the precursor of TXA₂. In human platelets, TXA₂ provides a mechanism for amplifying the activation signal due to being synthesized and released in response to several platelet agonists and, in turn, induces an irreversible aggregation.

In short, the therapeutics employed in individuals with Systemic Arterial Hypertension is multiple, using pharmaceutical combinations in order to decrease blood pressure in spite of, in many cases, the side effects that are presented; however, it is of interest to use combinations that present a double effect and minimize the adverse reactions. With these object, tests were made by using the Amlodipine/Acetyl salicylic acid combination, with the aim to evaluate its effectiveness in antihypertensive response ratios, as well as its synergy and tolerance by evaluating the adverse effects, using fewer doses of ingredients, in addition to its effect as platelet aggregation inhibitor.

US 2003099724 A1 discloses a composition useful for the prevention and retardation of diabetic retinopathy, comprising magnesium carbonate, amlodipine besylate, amino guanidine, acetyl salicylic acid (aspirin), and ascorbic acid (Vitamin C).

The document FOLTS J.D.: "Inhibition of platelet activity in vivo by amlodipine alone and combined with aspirin". INTERNATIONAL JOURNAL OF CARDIOLOGY, vol. 62, no. SUPPL. 2, 1997, pages S111 - S117 reports on studies that investigated the activity of combinations of amlodipine and aspirin versus amlodipine alone on platelet activity in vivo.

WO 2004/080488 discloses compositions that comprise acetyl salicylic acid, an HMG-Co-A reductase inhibitor and an antihypertensive agent, and the examples disclose compositions comprising acetyl salicylic acid and amlodipine. The compositions are useful for the prevention of cardiovascular disorders in patients running a high risk of suffering from a cardiovascular disorder.

WO 01/76632 discloses compositions for the treatment or the reduction of the risk of cardiovascular disease, being high blood pressure one risk factor. This document discloses compositions which comprise aspirin and amlodipine.

### BRIEF DESCRIPTION OF THE INVENTION

The present invention describes a pharmaceutical combination comprising a combination of amlodipine and acetyl salicylic acid, for pharmaceutical use of the composition in the treatment of systemic arterial Hypertension and prevention of ischemic vascular events; the composition produces a combined product with improved properties and a synergistic effect.

In the conducted tests, one of the Amlodipine salts that provided good results was amlodipine besylate.

As of quantitative composition, it could be proved that a suitable concentration of amlodipine is that which is present in a range from 5 to 10 mg per dosage unit and, in a preferred embodiment, amlodipine is in a ratio of 5 mg per dosage unit. Good results were also achieved when Amlodipine was present in a concentration less than 100 mg per dosage unit.

An additional test allowed proving that a pharmaceutical composition in which acetyl salicylic acid is present in a concentration of 75 mg per dosage unit provided good results.

In the tests, calcium antagonists substances such as: nimodipine, nitrendipine, nicardipine, lercanidipine, nisoldipine, nifedipine, verapamile, diltiazem were not considered and it is not known how they could act.

Regarding the usage of the compositions, the invention is able to achieve a composition for the treatment of systemic arterial hypertension and for the prevention of ischemic vascular events.

### DETAILED DESCRIPTION OF THE INVENTION

In accordance to the invention, the pharmaceutical composition comprising a combination of amlodipine and acetyl salicylic acid was made, which combination produces a double effect: antihypertensive and platelet aggregation inhibiting effect, being better tolerated, and with fewer adverse effects, in patients with systemic arterial hypertension in risk of ischemic vascular events.

### EXAMPLE 1

A pilot study AMSSA was made in order to assess the usefulness of amlodipine in sick people with severe ischemic arterial hypertension.

The aim of such study was to assess the antihypertensive effectiveness of amlodipine in 40 individuals diagnosed with severe systemic arterial hypertension of difficult control and, as a secondary aim, to determine the platelet aggregation inhibiting effect with acetyl salicylic acid.

The platelet activation was measured by changes in light transmission of the platelet-rich plasma stimulated by a synthetic analogue of TXA₂, the U46619. All patients were treated with high doses of multiple hypotensive drugs; all of them presented side effects and a suitable control of hypertension was not achieved in any of them. After their inclusion, they were assessed every week for six months, then every two weeks until the control was achieved, with a total duration of the study of 36 weeks, during each medical visit a blood sample was collected in a tube containing 5% of citrate-dextrose and centrifuged at 1,000 rpm for 10 minutes.

### Results

The difference between the initial and final tension values resulted statistically significant (p<0.0001, Wilcoxon test). The cardioprotective effect was analyzed as a platelet aggregation inhibitor, which resulted statistically significant (p<0.0001) with the following benefits:

### Conclusions

In individuals with difficult controlled arterial hypertension in addition to cardiac complications proper to the organ, it is indispensable to be combined with platelet aggregation inhibiting therapy, the Amlodipine/Acetyl salicylic acid combination showed a wide range of safety and effectiveness.
□ Significantly reducing the risk of death for cardiovascular causes, myocardial infarction, ictus and refractory ischemia, as well as the anginal events of the transient ischemic vascular disease.
□ Significantly reducing platelet aggregation (involved in the genesis of atherosclerotic plaque)
□ Reducing the average number of drugs consumed from 2 to 1.
□ Absence of gastrointestinal adverse events.
□ Cardioprotective effect by the combination with acetyl salicylic acid.

### EXAMPLE 2

A comparative, double-blind study AMSAII was made, randomized to assess effectiveness and safety of the combination of amlodipine/acetyl salicylic acid as compared to amlodipine alone, in 20 high risk hypertensive patients, with electrocardiographic criteria of left ventricular hypertrophy in order to assess the decrease of cardiovascular complication risks in relation to treatment with amlodipine combined with acetyl salicylic acid, during a period of at least 6 months. Incidence of primary object (cardiovascular decease), ictus and myocardial infarction).

### Results

The results showed a significant decrease of 11% in individuals who received the Amlodipine/Acetyl salicylic acid combination, as compared to 15% of the group who received Amlodipine alone.

### Conclusions

The results of the study AMSAII indicate that amlodipine/acetyl salicylic acid exert a higher cardiovascular protection than amlodipine alone with reduction of blood pressure and reduction of platelet aggregation, higher tolerability and null adverse effects.

## Claims

1. A synergistic pharmaceutical composition for use in the treatment of systemic arterial hypertension **characterized in that** comprises: amlodipine in an amount of 5 mg per dosage unit and acetyl salicylic acid in an amount of 75 mg per dosage unit.

## Patentansprüche

1. Eine synergetische pharmazeutische Zusammensetzung zur Verwendung in der Behandlung systemischen Bluthochdrucks, **dadurch gekennzeichnet, dass** sie Folgendes umfasst: Amlodipin in einer Menge von 5 mg pro Dosiereinheit und Acetylsalicylsäure in einer Menge von 75 mg pro Dosiereinheit.

## Revendications

1. Composition synergique pharmaceutique pour une utilisation dans le traitement de l'hypertension artérielle systémique **caractérisée en ce qu'**elle comprend : de l'amlodipine à une quantité de 5 mg par unité posologique et de l'acide acétylsalicylique à une quantité de 75 mg par unité posologique.
